# EUROPÄISCHE PATENTANMELDUNG

(11) **EP 1 749 491 A2**
(43) Veröffentlichungstag der Anmeldung: **07.02.2007**
(21) Anmeldenummer: 06016231.0
(22) Anmeldetag: 03.08.2006
(51) Int. Cl.: A61B 17/88, A61B 17/34, A61F 2/46

(54) **Vorrichtung zur Behandlung von Wirbelkörpern**

(30) Priorität: 03.08.2005 US 195898
(71) Anmelder: Haberland, Nils, 60435 Frankfurt (DE); Weber, Helmut, 78576 Emmingen-Liptingen (DE)
(72) Erfinder: Haberland, Nils, 60435 Frankfurt (DE); Weber, Helmut, 78576 Emmingen-Liptingen (DE)
(74) Vertreter: Mussgnug, Bernd

(57) **Zusammenfassung**

Vorrichtung zur Behandlung von Wirbelkörpern (70) mit wenigstens einer Biopsie- und Zementkanüle (30), welche mit Knochenzement (60) befüllbar ist, und einem Stopfer (40), welcher in die Biopsie- und Zementkanüle einführbar ist, um den Knochenzement aus der Biopsie- und Zementkanüle herauszustößeln.

## Beschreibung

Die Erfindung betrifft ein Verfahren und eine Vorrichtung zur Behandlung von Wirbelkörpern.

Wirbelkörpererkrankungen wie osteoporotische Wirbelkörperfrakturen, metastatisch veränderte Wirbelkörper oder Hämangiomwirbel werden durch bekannte minimal-invasive Zementierungstechniken behandelt, welche den pathologisch veränderten Wirbelkörper stabilisieren und die mit den Erkrankungen eingehenden Schmerzen lindern.

Das herkömmliche Verfahren der Vertebroplastie beinhaltet die perkutane dorsale Einbringung von flüssigem Knochenzement unter Druck in den pathologisch veränderten Wirbelkörper, welcher durch entsprechende Lagerung des Patienten gegebenenfalls repositioniert wird. Aufgrund des flüssigen Zements und der Verwendung von Druck kommt es bei diesem Verfahren jedoch regelmäßig zu Austritt von Knochenzement. Tritt dieser in den Wirbelkanal ein, sind neurologische Ausfälle bis hin zur Querschnittslähmung die Folge. Weiterhin können Zementlungenembolien auftreten, falls der Knochenzement in das venöse System eintritt.

Der Erfindung liegt daher die Aufgabe zugrunde, ein Verfahren und eine Vorrichtung bereitzustellen, mit welchen eine verbesserte Durchführung des vertebroplastischen Verfahrens möglich ist.

Die Aufgabe wird gelöst durch das erfindungsgemäße Verfahren zur Behandlung von Wirbelkörpern gemäß Anspruch 1 und die erfindungsgemäße Vorrichtung zur Durchführung des Verfahrens gemäß Anspruch 10.

Vorteilhafte Ausgestaltungen und Weiterbildungen der Erfindung sind in den Unteransprüchen angegeben.

Das erfindungsgemäße Verfahren zur Behandlung eines Wirbelkörpers weist folgenden Schritte auf: Zunächst wird eine Trokarhülse unter Zuhilfenahme eines Trokars durch die Haut in den Wirbelkörper eingebracht. Der Trokar wird anschließend entfernt. Anschließend wird eine Biopsie- und Zementkanüle in die Trokarhülse eingeführt, die Spongiosa in die Biopsie- und Zementkanüle eingezogen und die Biopsie- und Zementkanüle wieder aus der Trokarhülse entfernt. Eine Biopsie- und Zementkanüle, welche mit Knochenzement gefüllt ist, wird anschließend in die Trokarhülse eingeführt. Schließlich wird der Knochenzement in den Wirbelkörper eingeführt, indem ein Stopfer in die Biopsie- und Zementkanüle eingebracht wird, welcher den in der Biopsie- und Zementkanüle befindlichen Knochenzement am distalen Ende der Biopsie- und Zementkanüle herausdrückt.

Der Vorteil dieses Verfahrens liegt darin, dass im Gegensatz zum Einbringen des flüssigen Knochenzements mit einer Spritze, in welcher der Knochenzement mit Druck durch eine enge Nadel gepresst wird, vorliegend durch gezieltes dosiertes Einstößeln unter Verwendung des Stopfers Knochenzement in den Wirbelkörper eingebracht werden kann, ohne den Knochenzement mit hohem Druck in den Wirbelkörper forcieren zu müssen, so dass die Gefahr des Austritts von Knochenzement in den Wirbelkanal oder das venöse System verringert wird. Durch das Entfernen von zumindest Teilen der Spongiosa mittels einer Biopsie- und Zementkanüle wird ein Hohlraum in dem Wirbelkörper erzeugt, in welchen anschließend der Knochenzement eingefüllt werden kann.

Das Erzeugen eines Hohlraums, bei welchem zumindest Teile der Spongiosa aus dem Wirbelkörper herausgezogen wird, hat den Vorteil, dass beim Einfüllen des Knochenzements kein Druck auf den Wirbelkörper ausgeübt werden muss. Weiterhin können erkrankte Teile der Spongiosa vollständig entfernt werden und verbleiben nicht im Wirbelkörper. Insbesondere wird dadurch, dass zunächst ein Hohlraum erzeugt wird, eine gute Verteilung des Knochenzementes im Bereich des Wirbelkörpers mit guter biomechanischer Verankerung erreicht.

Bei einer besonders vorteilhaften Weiterbildung des erfindungsgemäßen Verfahrens wird der Knochenzement in zähflüssigem Zustand in den Wirbelkörper eingebracht. Gerade die Verarbeitung von flüssigem Knochenzement unter hohem Druck stellt bei den bekannten Verfahren der Vertebroplastie das Hauptrisiko dar, da der flüssige Knochenzement besonders leicht in den Wirbelkanal oder das venöse System austreten kann. Die Verwendung einer Biopsie- und Zementkanüle, aus welcher der Knochenzement unter Zuhilfenahme eines Stopfers eingestößelt wird, ermöglicht jedoch die Verwendung von zähflüssigem Knochenzement. Der zähflüssige Knochenzement kann sich ideal im Wirbelkörper verteilen und es besteht ein sehr geringes Komplikationsrisiko hinsichtlich des Zementaustritts aus dem Wirbelkörper.

Vorzugsweise werden zwei Biopsie- und Zementkanülen durch zwei Trokarhülsen in den Wirbelkörper eingebracht. Dadurch wird ein größerer Bereich des Innenraums des Wirbelkörpers erreicht, aus welchem die Spongiosa entfernt und Knochenzement eingefüllt wird, wodurch der Wirbelkörper besser stabilisiert wird. Um den Innenraum des Wirbelkörpers besonders effektiv mit zwei Trokarhülsen erreichen zu können, werden bevorzugt zwei Trokarhülsen symmetrisch in den Wirbelkörper eingebracht.

Bevorzugt werden die wenigstens eine Trokarhülse transpedikulär in den Wirbelkörper eingebracht, da dies den günstigsten Zugang zum Wirbelkörper und zum Innenraum des Wirbelkörpers ermöglicht. Insbesondere werden dadurch Beschädigungen der Nervenwurzel, die eventuell bei einem extrapedikulären Zugang auftreten können, vermieden.

Vorzugsweise weist der Stopfer Markierung auf, die dem in den Wirbelkörper einzubringenden Volumen an Knochenzement entsprechen. Wird der Stopfer Schritt für Schritt in die Biopsie- und Zementkanüle eingebracht, kann somit anhand der am Außenumfang des Stopfers angeordneten Markierung bestimmt werden, wie viel Knochenzement bereits am distalen Ende der Biopsie- und Zementkanüle ausgetreten und in den Wirbelkörper eingefüllt wurde.

Bevorzugt wird das Verfahren zumindest abschnittsweise unter Computernavigation und/oder Röntgenbildwandlerkontrolle durchgeführt, um die Positionierung des Trokars und der Trokarhülse und/oder die Befüllung mit Knochenzement verfolgen und gegebenenfalls korrigieren zu können. Bevorzugt erfolgt daher das gesamte Verfahren unter Computernavigation und/oder Röntgenbildwandlerkontrolle.

Die erfindungsgemäße Vorrichtung zur Behandlung eines Wirbelkörpers, insbesondere zur Verwendung in dem erfindungsgemäßen Verfahren, weist wenigstens eine Biopsie- und Zementkanüle, welche mit Knochenzement befüllbar ist, und einen Stopfer, welcher in die Biopsie- und Zementkanüle einführbar ist, auf. Wird der Stopfer Schritt für Schritt in die Biopsie- und Zementkanüle eingeführt, wird der Knochenzement aus der Biopsie- und Zementkanüle vorsichtig herausgestößelt. Dies ermöglicht ein dosiertes Einstößeln des Knochenzements, bei welchem insbesondere auch zähflüssiger Knochenzement in das Innere des Wirbelkörpers eingebracht werden kann, was das Risiko eines Zementaustritts und damit verbundenen Schäden gegenüber dem Einbringen von flüssigem Knochenzement mittels einer forcierten Injektionstechnik deutlich verringert.

Vorzugsweise weist der Stopfer einen zylindrischen Stab mit einem am proximalen Ende angeordneten Knauf auf, wobei die Länge des zylindrischen Stabs der Gesamtlänge der Biopsie- und Zementkanüle entspricht. Ist der Stopfer somit vollständig in die Biopsie- und Zementkanüle eingeführt, schließt das distale Ende des zylindrischen Stabs mit dem distalen Ende der Biopsie- und Zementkanüle ab. Ein Einführen des Stopfers über die Länge der Biopsie- und Zementkanüle hinaus, was zu einer Schädigung des Wirbelkörpers führen könnte, wird somit zuverlässig vermieden.

Vorzugsweise entspricht der Außendurchmesser des zylindrischen Stabs des Stopfers dem Innendurchmesser der Biopsie- und Zementkanüle. Wird somit der Stopfer in die Biopsie- und Zementkanüle eingeführt, wird der in diesem Volumen befindliche Knochenzement vollständig verschoben, so dass eine exakte Dosierung der Menge an Knochenzement, welche in den Innenraum des Wirbelkörpers eingefüllt wird, möglich ist. Insbesondere kann beim Einführen des Stopfers in die Biopsie- und Zementkanüle kein Knochenzement am proximalen Ende der Biopsie- und Zementkanüle austreten. Die exakte Dosierung wird weiterhin dadurch ermöglicht, dass der Stopfer am Außenumfang Markierungen aufweist, die dem in den Wirbelkörper einzubringenden Volumen an Knochenzement entsprechen.

Bei einer vorteilhaften Weiterbildung der Erfindung weist auch die Biopsie- und Zementkanüle am Außenumfang äquidistante Markierungen auf. Diese dienen dazu festzustellen, wie weit die Biopsie- und Zementkanüle in die Trokarhülse eingeschoben wurde, um zu verhindern, dass die Biopsie- und Zementkanüle zu weit in den Innenraum des Wirbelkörpers eingeschoben wird, was zu Beschädigungen des Wirbelkörpers führen könnte.

Vorzugsweise werden zwei Biopsie- und Zementkanülen verwendet, um den Zugang zum Innenraum des Wirbelkörpers zu verbessern und einen größeren Bereich des Innenraums des Wirbelkörpers mit Knochenzement gleichmäßiger ausfüllen zu können.

Vorzugsweise weist die Vorrichtung einen Trokar und eine Trokarhülse auf. Diese sind vorzugsweise in ihren Abmessungen auf die Biopsie- und Zementkanüle abgestimmt, um einen optimalen Eingriff zu ermöglichen.

Bevorzugt weist der Trokar einen zylindrischen Stab mit einem am proximalen Ende angeordneten Knauf und einer am distalen Ende angeordneten Trokarspitze auf, wobei die Länge des zylindrischen Stabs ohne die Trokarspitze der Gesamtlänge der Trokarhülse entspricht. Der Trokar kann somit so in die Trokarhülse eingeschoben werden, dass nur die Trokarspitze über die Trokarhülse hinausragt. In diesem Zustand können Trokar und Trokarhülse besonders einfach in den Wirbelkörper eingeführt werden.

Bei einer vorteilhaften Ausgestaltung der Erfindung entspricht der Außendurchmesser des zylindrischen Stabs des Trokars dem Innendurchmesser der Trokarhülse. Dadurch wird ein bündiger Abschluss zwischen dem zylindrischen Stab des Trokars und der Trokarhülse erreicht, wodurch sich der Trokar einschließlich der Trokarhülse einfacher in den Wirbelkörper einbringen lässt.

Vorzugsweise weist die Trokarhülse am Außenumfang Markierungen auf. An diesen Markierungen kann abgelesen werden, wie weit die Trokarhülse in den Wirbelkörper eingeführt wurde.

Bevorzugt entspricht der Innendurchmesser der Trokarhülse dem Außendurchmesser der Biopsie- und Zementkanüle. Somit kann auch die Biopsie- und Zementkanüle nahezu spielfrei in die Trokarhülse eingebracht werden. Weiterhin werden die Außendimensionen der Trokarhülse so gering wie möglich gehalten, um einen möglichst schonenden Eingriff in den Wirbelkörper zu ermöglichen.

Bei einer bevorzugten Weiterbildung der Erfindung ist der Trokar computernavigationsfähig. Dazu weist er insbesondere Anschlüsse für einen Navigationsadapter auf. Dies ermöglicht ein besonders sicheres Einbringen des Trokars in den Wirbelkörper, wobei Beschädigungen des Wirbelkörpers weitestgehend vermieden werden.

Vorzugsweise weist der Trokar an seinem Knauf einen Stahlring, vorzugsweise mit Anschlüssen für eine Röntgenzieleinrichtung, auf. Ansonsten besteht der Knauf aus einem Material, welches für Röntgenstrahlen durchlässig ist. Damit kann das Einbringen des Trokars einschließlich der Trokarhülse unter Röntgenbildwandlerkontrolle geschehen, wobei insbesondere durch das Verwenden einer Röntgenzieleinrichtung eine genaue Positionierung des Trokars einschließlich der Trokarhülse ermöglicht wird und Beschädigungen am Wirbelkörper vermieden werden.

Ein Ausführungsbeispiel der Erfindung wird anhand der nachfolgenden Figuren ausführlich erläutert. Es zeigt
- Figur 1a: eine Seitenansicht durch einen Lendenwirbel, bei welchem eine Trokarhülse transpedikulär eingebracht wurde,
- Figur 1b: einen Schnitt gemäß Linie A-A in Figur 1a,
- Figur 2a: eine Seitenansicht durch einen Lendenwirbel, bei welchem eine Trokarhülse extrapedikulär eingebracht wurde,
- Figur 2b: einen Schnitt gemäß Linie B-B in Figur 2a,
- Figur 3a: eine Seitenansicht durch einen Brustwirbel, bei welchem eine Trokarhülse transpedikulär eingebracht wurde,
- Figur 3b: einen Schnitt gemäß Linie C-C in Figur 3a,
- Figur 4a: eine Seitenansicht durch einen Brustwirbel, bei welchem eine Trokarhülse extrapedikulär eingebracht wurde,
- Figur 4b: einen Schnitt gemäß Linie D-D in Figur 4a,
- Figur 5: eine Ansicht eines Trokars,
- Figur 6: eine Ansicht einer Trokarhülse,
- Figur 7: eine schematische Darstellung des Verfahrensschritts des Einbringens einer Trokarhülse in den Wirbelkörper,
- Figur 8: eine schematische Darstellung des Verfahrensschritts des Einbringens einer weiteren Trokarhülse in den Wirbelkörper,
- Figur 9: eine schematische Darstellung des Verfahrensschritts des Einbringens zweier Biopsie- und Zementkanülen in die Trokarhülsen,
- Figur 10: eine schematische Darstellung des Verfahrensschritts des Einziehens von Spongiosa in die Biopsie- und Zementkanülen,
- Figur 11: eine schematische Darstellung des Wirbelkörpers, bei welchem dir Spongiosa teilweise entfernt wurde,
- Figur 12: eine Ansicht der Biopsie- und Zementkanüle,
- Figur 13: eine Ansicht eines Stopfers,
- Figur 14: eine schematische Darstellung des Verfahrensschritts des Entfernens des Biopsiepräparats aus der Biopsie- und Zementkanüle,
- Figur 15: eine schematische Darstellung des Verfahrensschritts des Füllens von Knochenzement in eine Spritze,
- Figur 16: eine schematische Darstellung des Verfahrensschritts des Füllens von Knochenzement in die Biopsie- und Zementkanüle,
- Figur 17: eine schematische Darstellung der Biopsie- und Zementkanüle mit aufgesetztem Stopfer,
- Figur 18: eine schematische Darstellung der in die Trokarhülsen eingeführten Biopsie- und Zementkanülen mit aufgesetzten Stopfern,
- Figur 19: eine schematische Darstellung des Verfahrensschritts des Einfüllens des Knochenzements in den Wirbelkörper,
- Figur 20: eine schematische Darstellung des Wirbelkörpers nach dem Einfüllprozess gemäß Figur 19,
- Figur 21: eine schematische Darstellung des Wirbelkörpers mit entfernten Biopsie- und Zementkanülen,
- Figur 22: eine schematische Darstellung des Wirbelkörpers mit eingefülltem Knochenzement,
- Figur 23: eine Seitenansicht eines Trokars gemäß einem Ausführungsbeispiel der erfindungsgemäßen Vorrichtung,
- Figur 24: eine Seitenansicht einer Trokarhülse gemäß einem Ausführungsbeispiel der erfindungsgemäßen Vorrichtung,
- Figur 25: eine Seitenansicht einer Biopsie- und Zementkanüle gemäß einem Ausführungsbeispiel der erfindungsgemäßen Vorrichtung und
- Figur 26: eine Seitenansicht eines Stopfers gemäß einem Ausführungsbeispiel der erfindungsgemäßen Vorrichtung.

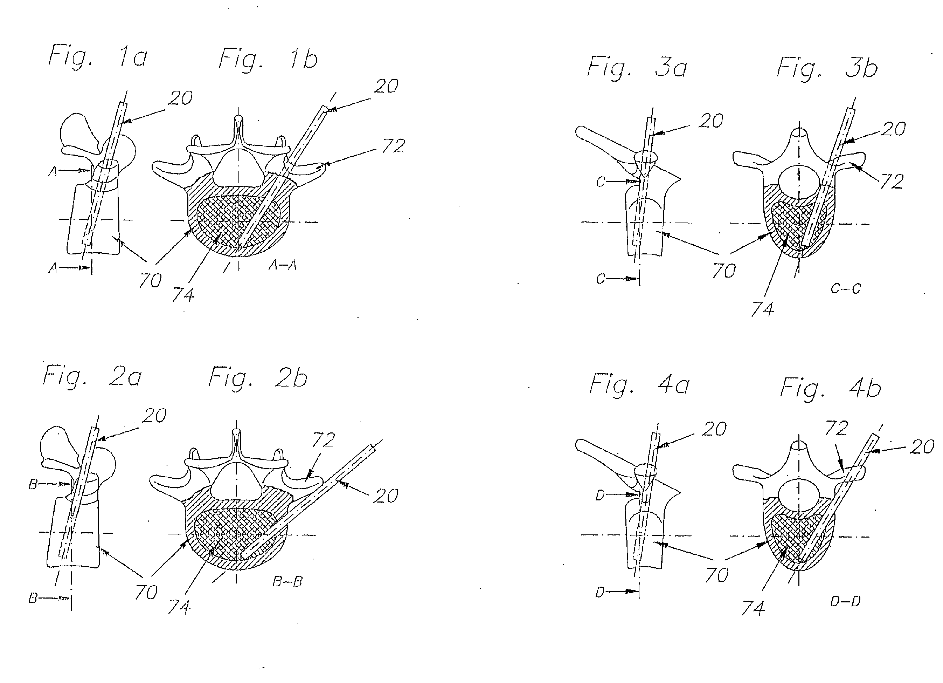

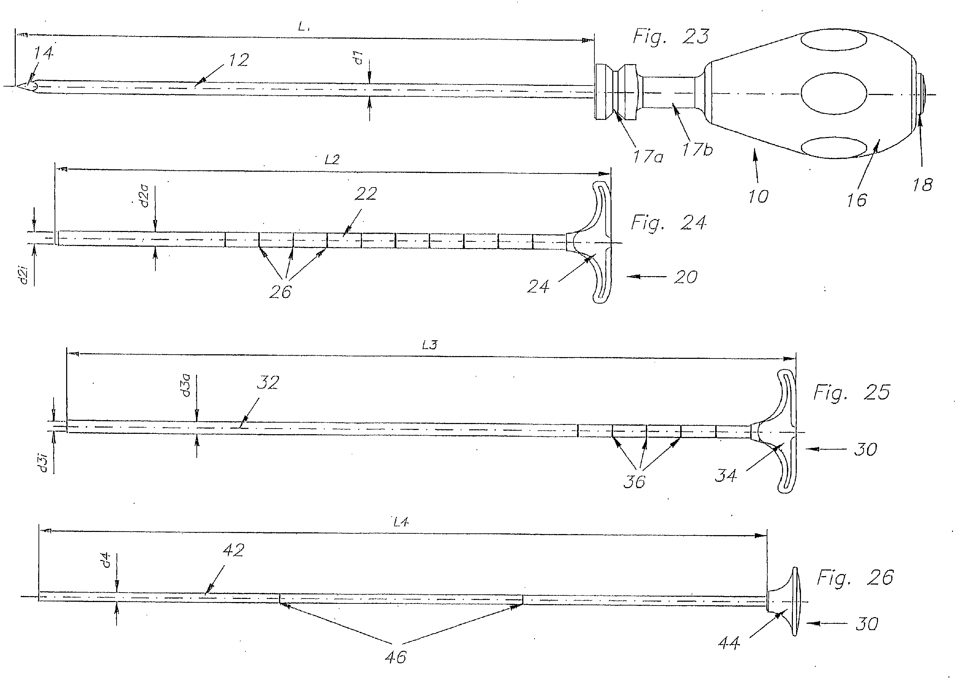

Zunächst soll ein Ausführungsbeispiel der erfindungsgemäßen Vorrichtung anhand der Figuren 23 bis 26 ausführlich erläutert werden, um anschließend die Anwendung der erfindungsgemäßen Vorrichtung in dem erfindungsgemäßen Verfahren anhand der Figuren 1 bis 22 zu beschreiben.

Figur 23 zeigt eine Seitenansicht eines Trokars 10 mit einem zylindrischen Stab 12 und einem an dem proximalen Ende des Stabes 12 angeordneten Knauf 16 aus einem Material, das für Röntgenstrahlen durchlässig ist. Das distale Ende des Stabes 12 weist eine Trokarspitze 14 auf. Der Stab 12 weist einschließlich der Trokarspitze 14 eine Länge L1 von etwa 170 mm, wobei die Trokarspitze 14 eine Länge von etwa 5 mm hat, und einen Außendurchmesser d1 von etwa 3,5 mm auf. Am Übergang zwischen dem zylindrischen Stab 12 und dem Knauf 16 befinden sich zwei Anschlüsse 17a, 17b für nicht dargestellte Navigationsadapter. Am Knaufende befindet sich ein Stahlring 18, der als Röntgenzieleinrichtung dient.

Der Trokar 10 ist in eine Trokarhülse 20 einführbar, welche in einer Seitenansicht in Figur 24 dargestellt ist. Die Trokarhülse 20 weist eine Hülse 22 und einen am proximalen Ende der Hülse 22 angeordneten Griff 24 aus ebenfalls strahlendurchlässigem Material auf. Die Hülse 22 wird durch ein zylindrisches Rohr mit einem Außendurchmesser d2a und einem Innendurchmesser d2i gebildet. Dabei setzt sich der Innenhohlraum der Hülse 22 durch den Griff 24 fort, so dass der Trokar 10 durch den Griff 24 in die Hülse 22 der Trokarhülse 20 eingeführt werden kann. Der Außendurchmesser d2a der Hülse 22 beträgt etwa 4,2 mm. Der Innendurchmesser d2i der Hülse 22 entspricht etwa dem Außendurchmesser d1 des Stabes 12 des Trokars 10, ist jedoch geringfügig größer, damit sich der Trokar 10 einführen lässt, und beträgt etwa 3,6 mm. Die Gesamtlänge L2 der Trokarhülse 20 beträgt 165 mm und entspricht somit etwa der Länge L1 des zylindrischen Stabs 12 des Trokars 10 ohne der Länge der Trokarspitze 14. Wird der Trokar 10 durch den Griff 24 in die Trokarhülse 20 eingeführt, bis der Knauf 16 an dem Griff 24 anschlägt, ragt somit nur die Trokarspitze 14 am distalen Ende der Trokarhülse 20 aus der Hülse 22 hervor.

Am Außenumfang der Trokarhülse 20 sind umlaufende Markierungen 26 angeordnet. Bevorzugt sind diese Markierungen äquidistant angeordnet. Im vorliegenden Ausführungsbeispiel beträgt der Abstand der Markierungen 26 etwa 10 mm. An diesen Markierungen 26 kann beim Einführen des Trokars 10 einschließlich der Trokarhülse 20 in den Körper des Patienten und insbesondere in den Wirbelkörper abgelesen werden, wie weit die Trokarhülse 20 bereits eingeführt wurde.

Figur 25 zeigt eine Seitenansicht einer Biopsie- und Zementkanüle 30 mit einer als zylindrisches Rohr ausgebildeten Hülse 32 und einem an deren proximalen Ende angeordneten Griff 34. Die Biopsie- und Zementkanüle 30 weist eine Gesamtlänge L3 von etwa 210 mm auf. Die Hülse 32 der Biopsie- und Zementkanüle 30 weist einen Außendurchmesser d3a und einen Innendurchmesser d3i auf. Dabei setzt sich der Innenhohlraum der Hülse 32 durch den Griff 34 fort. Der Außendurchmesser d3a der Biopsie- und Zementkanüle 30 beträgt etwa 3,5 mm und entspricht etwa dem Innendurchmesser d2i der Trokarhülse 20, ist jedoch geringfügig kleiner ausgebildet, damit die Biopsie- und Zementkanüle 30 in die Trokarhülse 20 eingeführt werden kann. Der Innendurchmesser d3i der Biopsie- und Zementkanüle 30 beträgt etwa 3,1 mm.

Die Biopsie- und Zementkanüle 30 weist ausgehend von dem am proximalen Ende der Hülse 32 angeordneten Griff 34 umlaufende Markierungen 36 auf, welche vorzugsweise äquidistant angeordnet sind. Bei dem vorliegenden Ausführungsbeispiel sind die Markierungen 36 in einem Abstand von etwa 10 mm angeordnet. Anhand der Markierungen 36 kann abgelesen werden, wie weit die Biopsie- und Zementkanüle 30 in die Trokarhülse 20 eingeschoben wurde. Die Länge L3 der Biopsie- und Zementkanüle 30 ist deutlich größer, beispielsweise etwa um ein Viertel der Länge L3 größer, als die Länge L2 der Trokarhülse 20. Dies ermöglicht, dass das distale Ende der Hülse 32 der Biopsie- und Zementkanüle 30 auch über das distale Ende der Hülse 22 der Trokarhülse 20 hinausragen kann, so dass die Biopsie- und Zementkanüle 30 weiter in den Wirbelkörper hineingeschoben werden kann als die Trokarhülse 20. An den Markierungen 36 kann dabei exakt abgelesen werden, wie weit das distale Ende der Hülse 32 der Biopsie- und Zementkanüle 30 über das distale Ende der Hülse 22 der Trokarhülse 20 hinausragt.

In Figur 26 ist ein Ausführungsbeispiel eines Stopfers 40 dargestellt, welcher einen zylindrischen Stab 42 der Länge L4 und einen sich am proximalen Ende des Stabes 42 anschließenden Knauf 44 aufweist. Der Stab 42 weist einen Außendurchmesser d4 auf, welcher etwa 3 mm beträgt und somit etwa dem Innendurchmesser d3i der Biopsie- und Zementkanüle 30 entspricht, jedoch geringfügig kleiner ausgebildet ist, damit sich der Stopfer 40 in die Biopsie- und Zementkanüle 30 einführen lässt.

Der Stopfer 40 weist an dem zylindrischen Stab 42 umlaufende Markierungen 46 auf, welche vorzugsweise äquidistant angeordnet sind. Im vorliegenden Ausführungsbeispiel sind diese ausgehend von dem distalen Ende des Stabs 42 im Abstand von etwa 70 mm angeordnet. Aufgrund des Außendurchmessers von etwa 3 mm kennzeichnen somit die Markierungen 46 im Abstand von 70 mm ein Volumen von etwa 0,5 cm³. Wird der Stopfer 40 in eine mit Knochenzement gefüllte Biopsie- und Zementkanüle 30 eingeschoben, kann somit anhand der Markierungen 46 das Volumen des am distalen Ende der Biopsie- und Zementkanüle 30 austretenden Knochenzements abgelesen werden. Wird beispielsweise der Stopfer 40 um zwei Markierungen 46 in die Biopsie- und Zementkanüle 30 eingeführt, tritt bei vollständig mit Knochenzement 60 gefüllter Biopsie- und Zementkanüle 30 ein Volumen von etwa 1 cm³ Knochenzement 60 am distalen Ende der Biopsie- und Zementkanüle aus und in den Wirbelkörper ein.

Die Länge L4 des Stabs 42 beträgt etwa 210 mm und entspricht somit etwa der Gesamtlänge L3 der Biopsie- und Zementkanüle 30. Somit kann der Stopfer 40 vollständig durch den Griff 34 in die Biopsie- und Zementkanüle 30 eingeschoben werden, bis der Knauf 44 des Stopfers 40 an dem Griff 34 der Biopsie- und Zementkanüle 30 anschlägt, und das distale Ende des Stopfers 40 schließt in dieser Position bündig mit dem distalen Ende der Biopsie- und Zementkanüle 30 ab. Dadurch ist sichergestellt, dass das vollständige Volumen an Knochenzement aus der Biopsie- und Zementkanüle 30 in das Innere des Wirbelkörpers entleert werden kann.

Anhand der Figuren 1 bis 22 wird im Folgenden die Verwendung des Ausführungsbeispiels der erfindungsgemäßen Vorrichtung mit dem Trokar 10, der Trokarhülse 20, der Biopsie- und Zementkanüle 30 und dem Stopfer 40 in dem erfindungsgemäßen Verfahren beschrieben.

In den Figuren 1a bis 4b ist jeweils ein Wirbelkörper 70 mit den Pedikeln 72 in der Seitenansicht (Figuren 1a, 2a, 3a und 4a) sowie einem Vertikalschnitt (Figuren 1b, 2b, 3b und 4b) dargestellt. Bei dem in den Figuren 1a bis 2b dargestellten Wirbelkörper 70 handelt es sich um einen Lendenwirbel, während in den Figuren 3a bis 4b ein Brustwirbel dargestellt ist. Die Figuren 1a bis 4b zeigen, wie die Trokarhülse 20 in den Wirbelkörper 70 eingebracht werden kann. In den Figuren 1a, 1b, 3a und 3b ist dabei der transpedikuläre Zugang, in den Figuren 2a, 2b, 4a und 4b der extrapedikuläre Zugang gezeigt. Bevorzugt wird im folgenden Verfahren die Trokarhülse 20 transpedikulär in den Wirbelkörper 70 eingebracht, da dies den Zugang zum Wirbelinnenraum vereinfacht. Insbesondere werden so Beschädigungen an der Nervenwurzel, die eventuell bei einem extrapedikulären Zugang auftreten können, vermieden.

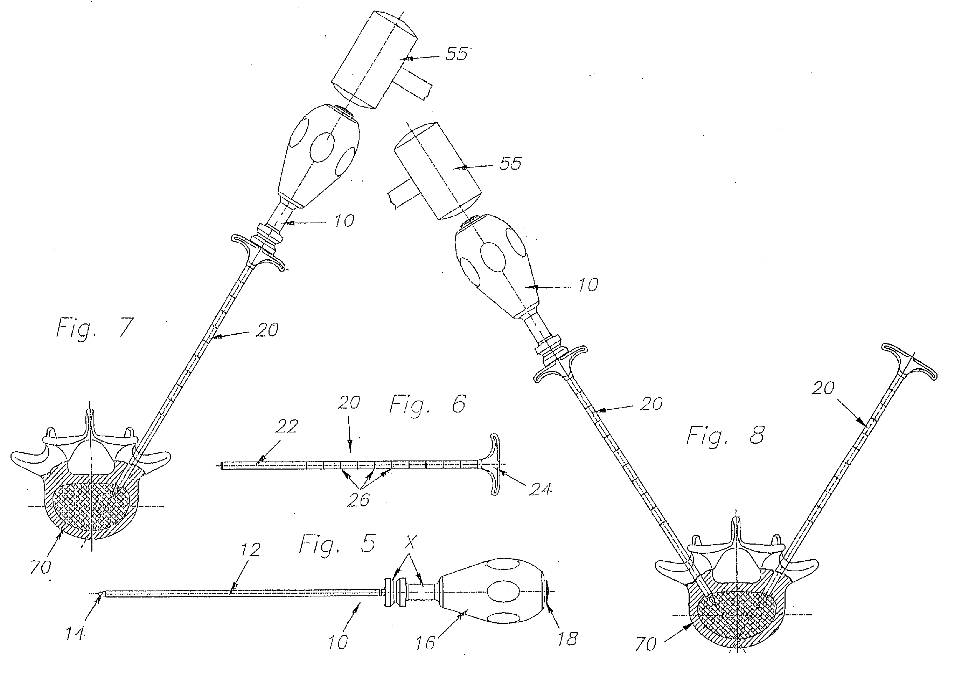

Figuren 5 und 6 zeigen die in einem ersten Verfahrensschritt verwendeten Instrumente, nämlich den Trokar 10 gemäß Figur 23 sowie die Trokarhülse 20 gemäß Figur 24. Der Trokar 10 wird in die Trokarhülse 20 eingeschoben, bis der Knauf 16 des Trokars 10 an dem Griff 24 der Trokarhülse 20 anschlägt, so dass die Trokarspitze 14 am distalen Ende der Trokarhülse 20 herausragt. Der Trokar 10 einschließlich der Trokarhülse 20 wird, wie in Figur 7 gezeigt, nach Freilegen des Zugangs zum Wirbelkörper 70 perkutan dorsal und transpedikulär durch vorsichtiges Einschlagen mit einem Hammer 55 in den Wirbelkörper 70 eingebracht, bis das distale Ende der Trokarhülse 20 gerade in den Innenraum des Wirbelkörpers 70 ragt. Diese Position kann anhand einer Computernavigation und/oder einer Röntgenbildwandlerkontrolle, wobei zusätzlich eine Röntgenzieleinrichtung zur Anwendung kommen kann, bestimmt werden. Vorteilhafterweise erfolgt das vollständige Verfahren unter Computernavigation und/oder Röntgenbildwandlerkontrolle, um jeden Verfahrensschritt kontrollieren zu können. Die Computernavigation erfolgt bevorzugt während des Einbringens des Trokars 10, um die richtige Positionierung des Trokars 10 zu überwachen. Im weiteren Verlauf des Verfahrens kann auf sie verzichtet werden. Weiterhin wird zumindest eine Röntgenbildwandlerkontrolle während des vollständigen Verfahrens verwendet. Sollten keine Daten für eine Computernavigation vorliegen, werden bevorzugt zwei Röntgenbildwandlerkontrollen verwendet.

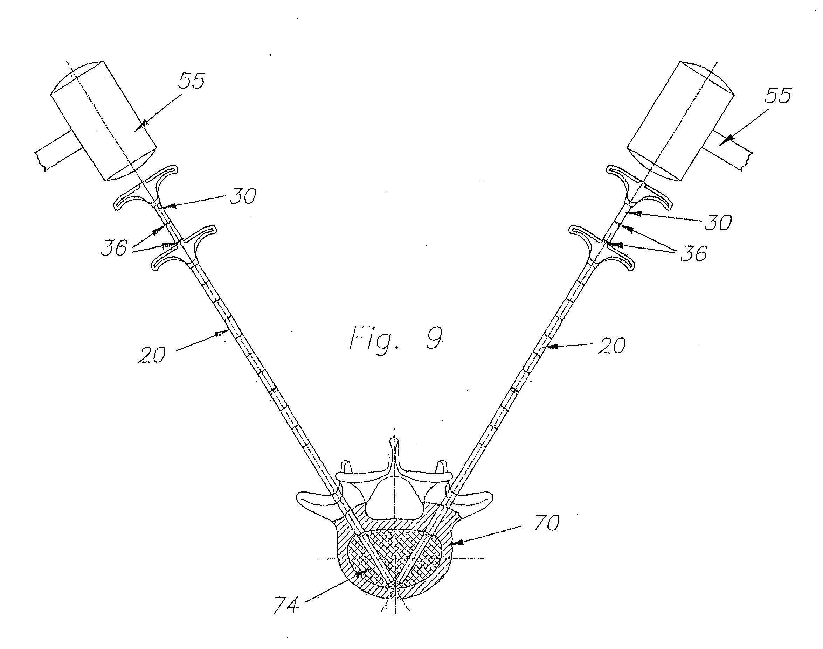

Wie in Figur 8 dargestellt, wird eine zweite Trokarhülse 20 symmetrisch zur ersten Trokarhülse 20 ebenfalls transpedikulär eingebracht.

Figur 9 zeigt, wie anschließend in jede der Trokarhülsen 20 jeweils eine Biopsie- und Zementkanüle 30 gemäß Figur 25 eingeführt wird. Durch leichtes Schlagen mit dem Hammer 55 werden jeweils rechts und links die Biopsie- und Zementkanülen 30 durch die Trokarhülsen 20 bis zur Vorderkante des Wirbelkörpers 70 eingetrieben. An den Markierungen 36 der Biopsie- und Zementkanüle 30 kann abgelesen werden, wie weit die Biopsie- und Zementkanüle 30 in die Trokarhülse 20 eingeschoben wurde. Da die Länge L3 Hülse 32 der Biopsie- und Zementkanüle 30 größer ist als die Länge L2 der Hülse 22 der Trokarhülse 20, kann das distale Ende der Biopsie- und Zementkanüle 30 über das distale Ende der Trokarhülse 20 hinausgeschoben werden. Anhand der Markierungen 36 wird kontrolliert, dass das distale Ende der Biopsie- und Zementkanüle 30 so weit über das distale Ende der Trokarhülse 20 hinausragt, dass es beinahe an der der Eintrittsöffnung in den Innenraum des Wirbelkörpers 70 gegenüberliegenden Innenfläche des Innenraums des Wirbelkörpers 70 anliegt (vgl. Figur 9). Die beiden Biopsie- und Zementkanülen 30 durchmessen somit im Wesentlichen den Innenraum des Wirbelkörpers 70.

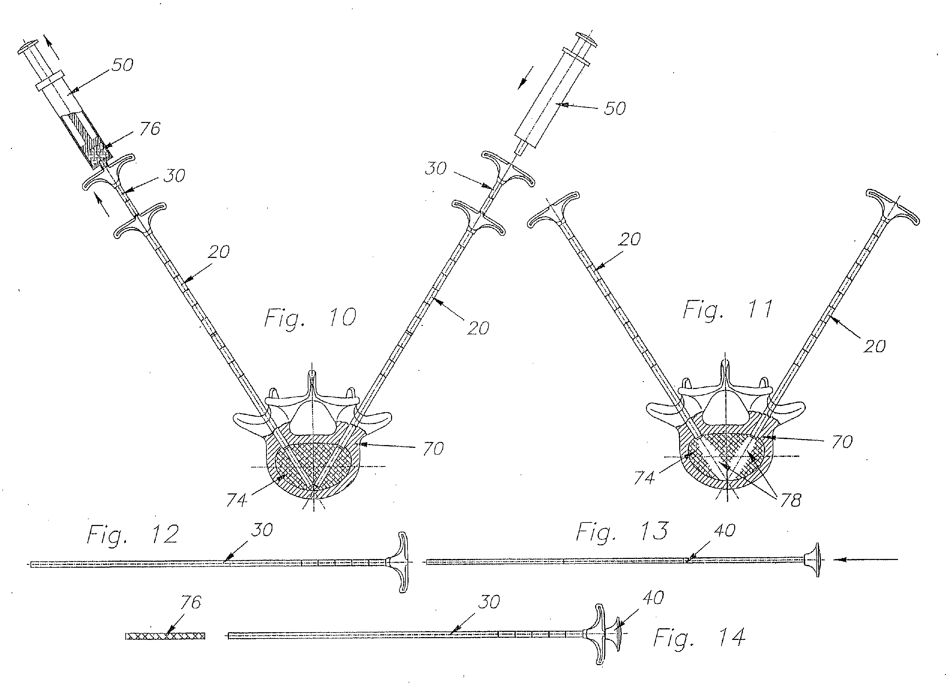

Figur 10 stellt dar, dass auf die beiden Griffe 34 der Biopsie- und Zementkanülen 30 eine Spritze 50 aufgesetzt wird, mit welcher unter Aspiration durch die Biopsie- und Zementkanülen 30 die Spongiosa 74 aus dem Innern des Wirbelkörpers 70 gezogen wird. Dabei werden gleichzeitig auch die Biopsie- und Zementkanülen 30 soweit aus den Trokarhülsen 20 gezogen, bis etwa das distale Ende der Biopsie- und Zementkanülen 30 mit dem distalen Ende der Trokarhülsen 20 abschließt, so dass zwei im Wesentlichen zylindrische Hohlräume 78 im Innern des Wirbelkörpers 70 entstehen (vgl. Figur 11). Die Hohlräume 78 haben dabei in einem Lendenwirbel beispielsweise eine Länge von etwa 25 mm und einen Durchmesser von etwa 3 mm. Die Erzeugung der Hohlräume 78 erleichtert das Einbringen des Knochenzements 60, welcher sich in den Hohlräumen ideal verteilen kann, wodurch das Risiko eines Knochenzementaustritts aus dem Wirbelkörper verringert wird. Anschließend werden die beiden Biopsie- und Zementkanülen 30 aus den Trokarhülsen 20 entfernt (vgl. Figur 11).

In den Figuren 12 bis 14 ist dargestellt, dass in die Biopsie- und Zementkanüle 30 der Stopfer 40 gemäß Figur 26 eingeführt wird, um das gewonnene Biopsiepräparat 76 aus der Biopsie- und Zementkanüle 30 heraus zu stoßen. Das Biopsiepräparat 76 kann gegebenenfalls für histologische Untersuchungen verwendet werden.

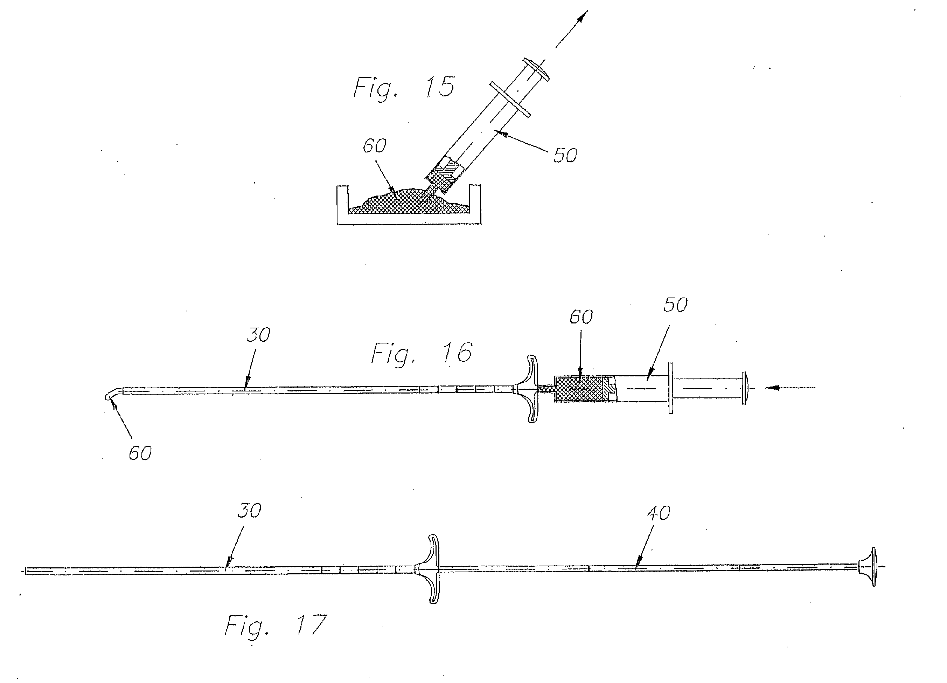

In Figur 15 ist dargestellt, wie Knochenzement 60 angerührt und in zähflüssigem Zustand in eine Spritze 50 eingezogen wird. Als Knochenzement 60 wird im Folgenden jedes Material bezeichnet, welches geeignet ist, einen Knochen, insbesondere einen Wirbelkörper, zu stabilisieren. Insbesondere können als Materialien Polymethylmetacrylat (PMMA) oder Kaliumphosphatzemente verwendet werden.

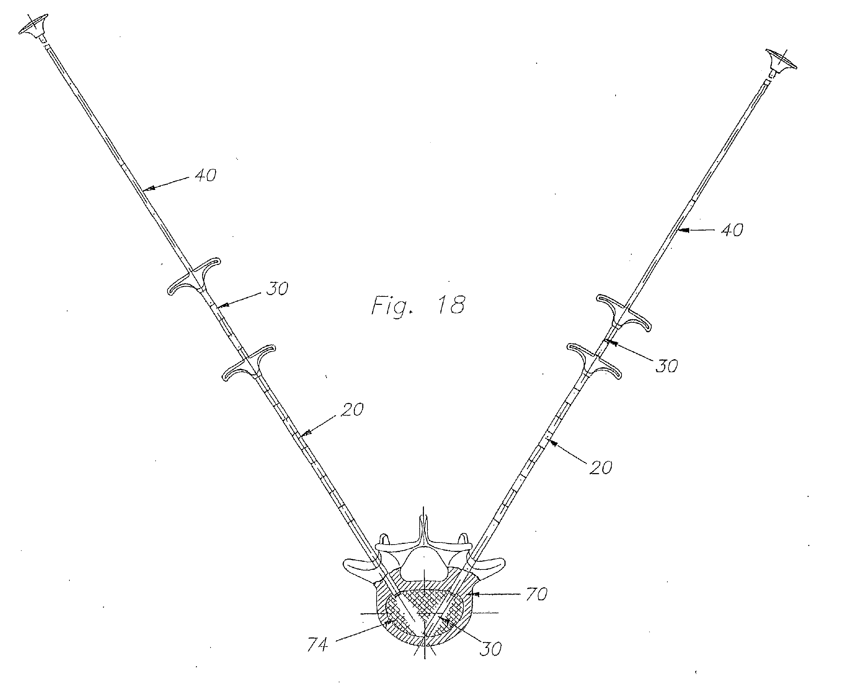

Anschließend wird der zähflüssige Knochenzement 60 in die Biopsie- und Zementkanüle 30 gefüllt. Dabei kann entweder die Biopsie- und Zementkanüle 30, mit welcher die Spongiosa 74 eingezogen wurde und welche anschließend gereinigt wurde, oder eine identisch ausgebildete weitere Biopsie- und Zementkanüle 30 verwendet werden. Damit sichergestellt ist, dass die Biopsie- und Zementkanüle 30 vollständig gefüllt ist, wird vorzugsweise solange mittels der Spritze 50 Knochenzement 60 eingefüllt, bis dieser am distalen Ende der Biopsie- und Zementkanüle 30 austritt (vgl. Figur 16). Dieser Überschuss an Knochenzement 50 wird anschließend entfernt. Schließlich wird der Stopfer 40 auf das proximale Ende der Biopsie- und Zementkanüle 30 aufgesetzt (vgl. Figur 17) und die Biopsie- und Zementkanüle 30 mit aufgesetztem Stopfer 40 in die Trokarhülse 20 eingeführt (vgl. Figur 18). Alternativ kann auch zuerst die Biopsie- und Zementkanüle 30 in die Trokarhülse 20 eingeführt und anschließend der Stopfer 40 auf das proximale Ende der Biopsie- und Zementkanüle 30 aufgesetzt werden. Die Biopsie- und Zementkanüle 30 wird wiederum so weit in die Trokarhülse 20 eingeführt, dass das distale Ende der Biopsie- und Zementkanüle 30 über das distale Ende der Trokarhülse 20 hinaus ragt und beinahe an der gegenüberliegenden Innenwand des Wirbelkörpers anliegt (vgl. Figur 18).

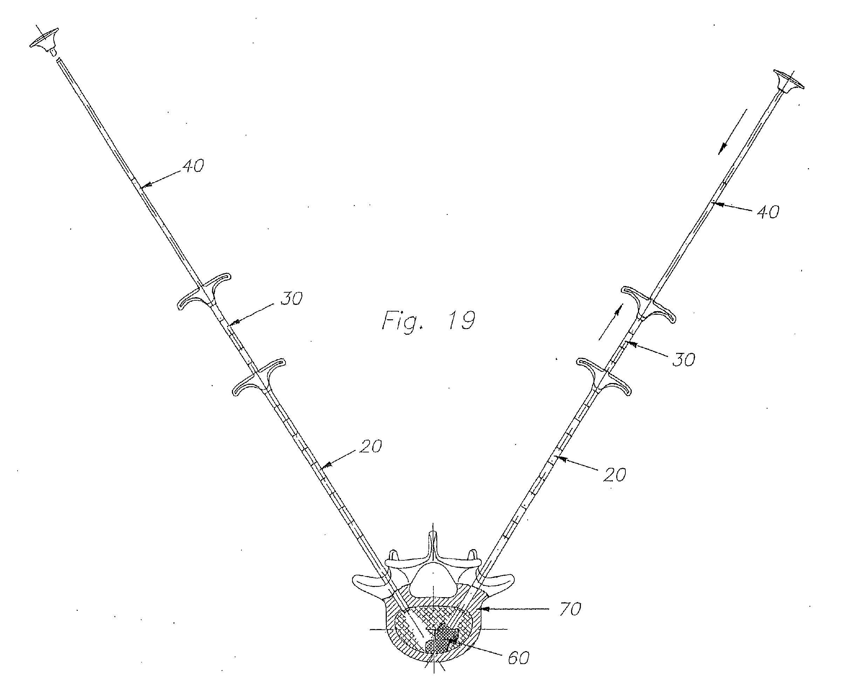

In Figur 19 ist nun der Verfahrensschritt des Befüllens des Innenraums des Wirbelkörpers 70 mit Knochenzement 60 dargestellt. Der Knochenzement 60 wird dadurch in den Wirbelkörper 70 eingefüllt, dass der Stopfer 40 langsam schrittweise in die Biopsie- und Zementkanüle 30 eingestößelt wird, während die Biopsie- und Zementkanüle 30 schrittweise aus dem Wirbelkörper 70 herausgezogen wird. Anhand der Markierungen 36 an der Biopsie- und Zementkanüle 30 kann überprüft werden, wie weit die Biopsie- und Zementkanüle 30 noch in den Innenraum des Wirbelkörpers 70 hineinragt, während anhand der Markierungen 46 an dem Stopfer 40 überprüft werden kann, wie viel Knochenzement 60 bereits in den Wirbelkörper 70 eingefüllt wurde. Die Biopsie- und Zementkanüle 30 wird soweit aus der Trokarhülse 20 herausgezogen, bis das distale Ende der Biopsie- und Zementkanüle 30 mit dem distalen Ende der Trokarhülse 20 abschließt. Insbesondere dieser Verfahrensschritt wird bevorzugt unter Röntgenbildwandlerkontrolle beobachtet, um das Einbringen des Knochenzements 60 zu jedem Zeitpunkt kontrollieren zu können und gegebenenfalls das Einfüllen sofort abbrechen zu können, falls Austritte von Knochenzement 60 aus dem Wirbelkörper 70 zu beobachten sind.

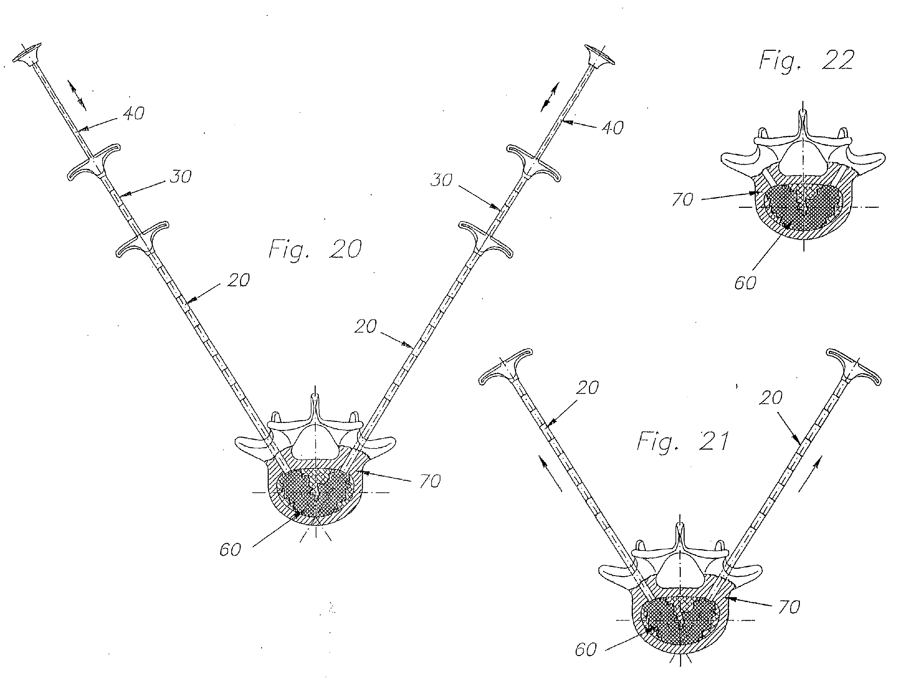

Auf die gleiche Weise wird auch über die zweite Trokarhülse und die zweite Biopsie- und Zementkanüle 30 Knochenzement 60 in den Wirbelkörper 70 eingefüllt (vgl. Figur 20), während die erste Biopsie- und Zementkanüle 30 in ihrer Position verbleibt. Wurde die nötige Menge an Knochenzement 60 eingefüllt, verbleiben die Trokarhülsen 20, die Biopsie- und Zementkanülen 30 sowie die Stopfer 40 solange im Wirbelkörper 70, bis der Knochenzement 60 vollständig abgebunden ist. Anschließend werden die Stopfer 40, die Biopsie- und Zementkanülen 30 sowie die Trokarhülsen 20 nacheinander entfernt (vgl. Figur 21).

In Figur 22 ist das abschließende Ergebnis der Operation dargestellt, welches den Wirbelkörper 70 mit eingebrachtem Knochenzement 60 darstellt. Durch das erfindungsgemäße Verfahren wird kann somit zunächst ein Hohlraum 78 erzeugt werden, in welchen zähflüssiger Knochenzement 60 aus der Biopsie- und Zementkanüle 30 unter Zuhilfenahme des Stopfers 40 ohne Druck eingestößelt werden kann, wodurch das Risiko eines Austritts von Knochenzement 60 aus dem Wirbelkörper 70 deutlich verringert werden kann.

### Bezugszeichenliste

- 10: Trokar
- 12: Stab
- 14.: Trokarspitze
- 16: Knauf
- 17a: Anschluss für Navigationsadapter
- 17b: Anschluss für Navigationsadapter
- 18: Stahlring

- 20: Trokarhülse
- 22: Hülse
- 24: Griff
- 26: Markierung

- 30: Biopsie- und Zementkanüle
- 32: Hülse
- 34: Griff
- 36: Markierung

- 40: Stopfer
- 42: Stab
- 44: Knauf
- 46: Markierung

- 50: Spritze
- 55: Hammer
- 60: Knochenzement

- 70: Wirbelkörper
- 72: Pedikel
- 74: Spongiosa
- 76: Biopsiepräparat
- 78: Hohlraum

- L1: Länge (des zylindrischen Stabs des Trokars)
- L2: Länge (der Trokarhülse)
- L3: Länge (der Biopsie- und Zementkanüle)
- L4: Länge(des zylindrischen Stabs des Stopfers)

- d1: Außendurchmesser (des zylindrischen Stabs des Trokars)
- d2a: Außendurchmesser (der Trokarhülse)
- d2i: Innendurchmesser (der Trokarhülse)
- d3a: Außendurchmesser (der Biopsie- und Zementkanüle)
- d3i: Innendurchmesser (der Biopsie- und Zementkanüle)
- d4: Außendurchmesser (des zylindrischen Stabs des Stopfers)

## Patentansprüche

1. Verfahren zur Behandlung eines Wirbelkörpers (70), mit folgenden Schritten:
- Einbringen eines Trokars (10) und einer Trokarhülse (20) in den Wirbelkörper (70),
- Entfernen des Trokars (10),
- Einführen einer Biopsie- und Zementkanüle (30) in die Trokarhülse,
- Einziehen von Spongiosa (74) in die Biopsie- und Zementkanüle (30)
- Entfernen der Biopsie- und Zementkanüle (30),
- Füllen einer Biopsie- und Zementkanüle (30) mit Knochenzement (60),
- Einführen der Biopsie- und Zementkanüle (30) in die Trokarhülse (20) und
- Einbringen des Knochenzements (60) in den Wirbelkörper (70) durch Einbringen eines Stopfers (40) in die Biopsie- und Zementkanüle (30).

2. Verfahren nach Anspruch 1,
**dadurch gekennzeichnet, dass** der Knochenzement (60) in zähflüssigem Zustand in den Wirbelkörper (70) eingebracht wird.

3. Verfahren nach einem der vorhergehenden Ansprüche,
**dadurch gekennzeichnet, dass** der Knochenzement (60) unter Verwendung einer Spritze (50) in die Biopsie- und Zementkanüle (30) gefüllt wird.

4. Verfahren nach einem der vorhergehenden Ansprüche,
**dadurch gekennzeichnet, dass** zwei Biopsie- und Zementkanülen (30) durch zwei Trokarhülsen (20) in den Wirbelkörper (70) eingebracht werden.

5. Verfahren nach Anspruch 4,
**dadurch gekennzeichnet, dass** die beiden Biopsie- und Zementkanülen (30) symmetrisch in den Wirbelkörper (70) eingebracht werden.

6. Verfahren nach einem der vorhergehenden Ansprüche,
**dadurch gekennzeichnet, dass** die wenigstens eine Trokarhülse (20) transpedikulär in den Wirbelkörper (70) eingebracht wird.

7. Verfahren nach einem der vorhergehenden Ansprüche,
**dadurch gekennzeichnet, dass** der Stopfer (40) Markierung (46) aufweist, die dem in den Wirbelkörper (70) einzubringenden Volumen an Knochenzement (60) entsprechen.

8. Verfahren nach einem der vorhergehenden Ansprüche,
**dadurch gekennzeichnet, dass** das Verfahren zumindest abschnittsweise unter Computernavigation und/oder Röntgenbildwandlerkontrolle erfolgt.

9. Verfahren nach einem der vorhergehenden Ansprüche,
**dadurch gekennzeichnet, dass** das Verfahren vollständig unter Computernavigation und/oder Röntgenbildwandlerkontrolle erfolgt.

10. Vorrichtung zur Behandlung von Wirbelkörpern (70) mit wenigstens einer Biopsie- und Zementkanüle (30), welche mit Knochenzement (60) befüllbar ist, und einem Stopfer (40), welcher in die Biopsie- und Zementkanüle (30) einführbar ist, um den Knochenzement (60) aus der Biopsie- und Zementkanüle (30) herauszustößeln.

11. Vorrichtung nach Anspruch 10,
**dadurch gekennzeichnet, dass** der Stopfer (40) einen zylindrischen Stab (42) mit einem am proximalen Ende angeordneten Knauf (44) aufweist, wobei die Länge (L4) des zylindrischen Stabs (42) der Gesamtlänge (L3) der Biopsie- und Zementkanüle (30) entspricht.

12. Vorrichtung nach Anspruch 10 oder 11,
**dadurch gekennzeichnet, dass** der Außendurchmesser (d4) des zylindrischen Stabs (42) des Stopfers (40) dem Innendurchmesser (d3i) der Biopsie- und Zementkanüle (30) entspricht.

13. Vorrichtung nach einem der Ansprüche 10 bis 12,
**dadurch gekennzeichnet, dass** der Stopfer (40) am Außenumfang Markierungen (46) aufweist, die in den Wirbelkörper (70) dem einzubringenden Volumen an Knochenzement (60) entsprechen.

14. Vorrichtung nach einem der Ansprüche 10 bis 13,
**dadurch gekennzeichnet, dass** die Biopsie- und Zementkanüle (30) am Außenumfang äquidistante Markierungen (36) aufweist.

15. Vorrichtung nach einem der Ansprüche 10 bis 14,
**dadurch gekennzeichnet, dass** die Vorrichtung zwei Biopsie- und Zementkanülen (30) aufweist.

16. Vorrichtung nach einem der Ansprüche 10 bis 15,
**dadurch gekennzeichnet, dass** die Vorrichtung einen Trokar (10) und eine Trokarhülse (20) aufweist.

17. Vorrichtung nach Anspruch 16,
**dadurch gekennzeichnet, dass** der Trokar (10) einen zylindrischen Stab (12) mit einem am proximalen Ende angeordneten Knauf (16) und einer am distalen Ende angeordneten Trokarspitze (14) aufweist, wobei die Länge (L1) des zylindrischen Stabs (12) ohne der Trokarspitze (14) der Gesamtlänge (L2) der Trokarhülse (20) entspricht.

18. Vorrichtung nach Anspruch 16 oder 17,
**dadurch gekennzeichnet, dass** der Außendurchmesser (d1) des zylindrischen Stabs (12) des Trokars (10) dem Innendurchmesser (d2i) der Trokarhülse (20) entspricht.

19. Vorrichtung nach einem der Ansprüche 16 bis 18,
**dadurch gekennzeichnet, dass** die Trokarhülse (20) am Außenumfang Markierungen (26) aufweist.

20. Vorrichtung nach einem der Ansprüche 16 bis 19,
**dadurch gekennzeichnet, dass** der Innendurchmesser (d2i) der Trokarhülse (20) dem Außendurchmesser (d3a) der Biopsie- und Zementkanüle (30) entspricht.

21. Vorrichtung nach einem der Ansprüche 16 bis 20,
**dadurch gekennzeichnet, dass** der Trokar (10) Anschlüsse (17a,17b) für einen Navigationsadapter aufweist.

22. Vorrichtung nach einem der Ansprüche 16 bis 21,
**dadurch gekennzeichnet, dass** der Trokar (10) an seinem Knauf (16) einen Stahlring (18) aufweist und der Knauf (16) im Übrigen aus Material besteht, das für Röntgenstrahlen durchlässig ist.

23. Verwendung der Vorrichtung nach einem der Ansprüche 10 bis 22 in dem Verfahren nach einem der Ansprüche 1 bis 9.
